# FASCICULE DE BREVET EUROPEEN

(11) **EP 2 943 451 B1**
(45) Date de publication et mention de la délivrance du brevet: **15.03.2017**
(21) Numéro de dépôt: 14703900.2
(22) Date de dépôt: 10.01.2014
(51) Int. Cl.: C07C 1/24

(54) **PROCÉDÉ DE PURIFICATION DU CO2 COMPRIS DANS UN FLUX ÉTHYLÈNE ISSU DE LA DÉSHYDRATATION DE L'ÉTHANOL**
VERFAHREN ZUR REINIGUNG DES CO2 IN EINEM ETHYLENSTROM BEI DER ENTWÄSSERUNG VON ETHANOL
METHOD FOR PURIFYING THE CO2 CONTAINED IN AN ETHYLENE STREAM RESULTING FROM THE DEHYDRATION OF ETHANOL

(30) Priorité: 14.01.2013 FR 1300066
(43) Date de publication de la demande: 18.11.2015
(73) Titulaire: IFP Énergies nouvelles, 92852 Rueil-Malmaison Cedex (FR); Total Research & Technology Feluy, 7181 Seneffe (BE)
(72) Inventeur: COUPARD, Vincent, 69100 Villeurbanne (FR); PLENNEVAUX, Thomas, 69002 Lyon (FR); FLEURIER, Stéphanie, 69007 Lyon (FR); VERMEIREN, Walter, 3530 Houtalen-Helchteren (BE); MINOUX, Delphine, 1400 Nivelles (BE); DE SMEDT, Philip, 9100 Sint-Niklaas (BE); ADAM, Cindy, 5100 Wierde (BE); NESTERENKO, Nicolai, B- (BE)
(86) Numéro de dépôt international: PCT/FR2014/050043
(87) Numéro de publication internationale: WO 2014/108647

(56) Documents cités:
- WO-A1-2011/076752
- WO-A2-2007/134415

## Description

### Domaine de l'invention

Cette invention concerne un procédé permettant d'éliminer le CO₂ du flux d'éthylène issu des réacteurs du procédé de déshydratation d'éthanol. Cette invention porte en particulier sur le cas ou la concentration en CO₂ est trop importante pour permettre d'envisager raisonnablement la solution de l'adsorption sur une masse de captation.

### État de la technique antérieure

La demande de brevet WO2013/014002 A1 décrit la section de purification (après séchage) d'un flux d'éthylène comprenant au maximum 1 % poids de composés oxygénés (alcools, diéthyléther, acides etc...), de l'éthane, CO, CO₂, H₂, CH₄ et C₃+. Le brevet décrit les étapes de distillation permettant de séparer l'éthane, les oxygénés et les C₃⁺. Il décrit en particulier une solution de séparation du CO₂ utilisant une masse d'adsorption sélective. Enfin, il décrit une distillation permettant de séparer les dernières impuretés du flux d'éthylène.

Ce brevet explique que la séparation du CO₂ utilisant une tour à la soude oblige à séparer l'acétaldéhyde en amont (bouchage possible de l'absorbeur à la soude en raison de la polymérisation basique de l'acétaldéhyde), contrairement à la solution de l'adsorption qui est plus simple à mettre en oeuvre.

Ce brevet ne mentionne pas de concentration maximale en CO₂ dans le flux d'éthylène non purifié. Seules des concentrations globales en impuretés sont mentionnées.

WO2007134415 divulgue un procédé de déshydratation d'un charge d'éthanol en éthylène comprenant le chauffage d'un mélange d'éthanol et d'eau recyclé de séparateur par échange de chaleur et la réaction du mélange dans un réacteur.

Le brevet US 6459009 concerne un procédé de déshydratation d'alcool en lit fluidisé. Il décrit une solution de purification et d'intégration thermique utilisant deux colonnes consécutives de trempe à l'eau. La première colonne permet de séparer l'eau de l'effluent et de récupérer de la chaleur permettant de préchauffer la charge du procédé. La seconde colonne permet d'obtenir de l'eau purifiée recyclée dans la première colonne et un flux d'oléfines comprenant entre 10 ppm poids et 10 % poids d'oxygénés. Ce brevet concerne les procédés de déshydrations d'oxygénés en général mais mentionne la réaction de déshydratation du méthanol en oléfine (Methanol To Olefin, ou MTO suivant la désignation anglaise) en exemple : la réaction de MTO étant exothermique alors que celle de la présente invention est endothermique, l'application de l'un à l'autre ne va pas de soi. Il n'est pas fait mention de la séparation du CO₂ ni de la possibilité d'avoir une zone de compression entre les deux étages de trempe.

La demande de brevet WO 2011/076752 décrit une séparation des oxygénés compris dans un flux d'hydrocarbures. Il cite les oléfines issus de la déshydratation du méthanol en exemple. Ce brevet décrit l'utilisation d'une colonne d'absorption utilisant un solvant capable de capter l'eau et les oxygénés (par exemple glycols, amines). Ce solvant est régénéré par stripage ou distillation. Le flux comprenant les oxygénés est lavé a l'eau afin d'en séparer d'une part, les oxygénés et d'autre part, un flux qui peut être recyclé dans la section réactionnelle comprenant 10% d'oxygénés.

Ce brevet décrit ensuite la séparation des oxygénés restant dans la phase gaz de l'absorption à l'aide d'un lavage à la soude permettant d'éliminer les composant acides.

Le procédé décrit a le désavantage d'utiliser deux colonnes de lavage et d'introduire un tiers-corps (le solvant). Il ne mentionne pas la séparation du CO₂, cet effet étant toutefois induit par la présence de la colonne à la soude.

À notre connaissance, il n'existe pas de document concernant une solution de traitement d'un flux d'éthylène chargé en CO₂ issu d'une section réactionnelle de déshydratation d'éthanol mettant en oeuvre une unique colonne de lavage à l'eau suivie d'une colonne d'absorption du CO₂ à l'aide d'un solvant chimique ou physique.

### Résumé et intérêt de l'invention

L'invention concerne un procédé de production d'éthylène à partir d'une charge éthanol comprenant au moins les étapes suivantes :
a) Une étape de déshydratation d'une charge de déshydratation comprenant ladite charge éthanol de manière à produire au moins un effluent éthylène, et au moins un effluent eau,
b) Une étape de condensation partielle dudit effluent éthylène par échange de chaleur avec au moins une source froide à une température comprise entre 20 et 50°C,
c) Une étape de séparation de phases dudit effluent éthylène partiellement condensé de manière à produire un effluent gaz et un effluent liquide,
d) Une étape de compression dudit effluent gaz issu de l'étape b) de manière à produire un effluent gaz comprimé suivi d'un refroidissement à une température comprise entre 10 et 50°C,
e) Une étape de lavage par la mise en contact dudit effluent gaz comprimé avec l'eau de lavage recyclée selon l'étape h) dans une colonne de lavage de manière à produire un effluent gaz lavé et un effluent eau usée,
f) Une étape d'absorption par la mise en contact dudit effluent gaz lavé avec au moins une solution absorbante dans une colonne d'absorption, ladite solution absorbante comprenant au moins un solvant choisi parmi le groupe constitué par les solvants chimiques et physiques, de manière à produire un effluent gaz purifié et un effluent solution absorbante usée,
g) Une étape de distillation dudit effluent eau usée issu de l'étape e) et, conjointement, dudit effluent eau issu de ladite étape de déshydratation de manière à produire au moins un distillat gaz, un distillat liquide, un effluent eau purifiée, et un résidu de distillation.
h) Une étape de recyclage d'au moins une partie dudit effluent eau purifiée issu de l'étape g) en amont de l'étape e) de lavage, ladite partie étant alors désignée sous le nom d'eau de lavage.

Les spécifications commerciales de l'éthylène de grade polymère imposent une concentration maximum en CO₂ de 0,5 ppm volumique.

Un intérêt de cette invention est de permettre d'augmenter la flexibilité du procédé de déshydratation en augmentant la quantité acceptable de CO₂ dans l'effluent du réacteur. Un autre intérêt de cette invention est de limiter les équipements nécessaires à la purification de l'effluent de déshydratation en intégrant la purification de l'eau de lavage et de l'eau de dilution de la réaction dans une unique distillation. Cette invention permet de plus d'éviter la présence d'équipements et la consommation d'utilités dédiées à la mise en oeuvre et à la régénération de masses de captation du CO₂.

### Description de l'invention

Cette invention concerne le traitement d'un effluent éthylène issu d'une étape de déshydratation de l'éthanol. La réaction de déshydratation des alcools s'accompagne de la formation de CO₂ dont la concentration dans l'effluent peut varier en fonction, par exemple, de la qualité de la charge, des conditions opératoires, et du catalyseur.

### Charge

Conformément à l'invention, la charge traitée dans le procédé de déshydratation est une charge éthanol.

Ladite charge éthanol est avantageusement une charge éthanol hydratée concentrée. On entend par charge éthanol hydratée concentrée une charge éthanol comprenant un pourcentage massique en éthanol supérieur ou égal à 35% poids. De préférence, ladite charge éthanol hydratée concentrée comprend un pourcentage massique en éthanol compris entre 35 et 99,7% poids.

Ladite charge éthanol comprend également avantageusement, en plus de l'eau, une teneur en alcools autres que l'éthanol, tels que par exemple le méthanol, le butanol et/ou l'isopentanol inférieure à 10 % poids, et de préférence inférieure à 5 % poids, une teneur en composés oxygénés autres que les alcools tels que par exemple les éthers, les acides, les cétones, les aldéhydes et/ou les esters inférieure à 1 % poids et une teneur en azote et en soufre, organique et minéral, inférieure à 0,5 % poids, les pourcentages poids étant exprimés par rapport à la masse totale de ladite charge.

Ladite charge éthanol traitée est éventuellement obtenue par un procédé de synthèse d'alcool à partir de ressources fossiles telles que par exemple à partir du charbon, du gaz naturel ou des déchets carbonés.

Ladite charge éthanol peut également avantageusement provenir de ressources non fossiles. De préférence, la charge éthanol traitée dans le procédé selon l'invention est une charge éthanol produite à partir de sources renouvelables issues de la biomasse, souvent appelée "bioéthanol". Le bioéthanol est une charge produite par voie biologique, de préférence par fermentation de sucres issus par exemple des cultures de plantes sucrières comme la canne à sucre (saccharose, glucose, fructose, et sucrose), des betteraves, ou encore des plantes amylacées (amidon) ou de la biomasse lignocellulosique ou de cellulose hydrolysée (glucose majoritaire et xylose, galactose), contenant des quantités variables d'eau.

Pour une description plus complète des procédés fermentaires classiques, on peut se reporter a l'ouvrage « Les Biocarburants, État des lieux, perspectives et enjeux du développement », Daniel Ballerini, Editions Technip.

Ladite charge éthanol peut également avantageusement être obtenue à partir de gaz de synthèse.

Ladite charge éthanol peut également avantageusement également être obtenue par hydrogénation des acides ou esters correspondants. Dans ce cas, l'acide acétique ou les esters acétiques sont avantageusement hydrogénés à l'aide d'hydrogène en éthanol. L'acide acétique peut avantageusement être obtenu par carbonylation du méthanol ou par fermentation des carbohydrates.

### Étape a) de déshydratation

Conformément à l'invention, une charge de déshydratation comprenant ladite charge éthanol subit une étape a) de déshydratation dans une section réactionnelle en suivant une technologie connue de l'homme du métier, telle que décrite par exemple dans les documents US 4,396,789, WO 2011/002699 et WO 2007/134415 ou tout autre technologie de déshydratation catalytique de l'éthanol en éthylène.

Ladite charge de déshydratation comprend également avantageusement un appoint d'eau constitué de l'eau de dilution issue de l'étape h) de recyclage et/ou d'un flux d'eau d'origine externe au procédé.

Cet appoint d'eau permet de compenser la purge faite classiquement sur le recyclage de l'eau de dilution, lorsque le procédé comprend un tel recyclage, dont le débit partiel en eau serait supérieur à la quantité d'eau produite dans la section réactionnelle. En effet, il peut être avantageux d'augmenter la purge du procédé, par exemple pour éliminer une quantité anormalement élevée de sels provenant de la charge éthanol. Cette option permet de traiter des charges éthanol plus sales (chargée en sels, cendres...) que celle considérée pour le design de l'unité.

Ladite étape a) de déshydratation produit au moins un effluent éthylène et au moins un effluent eau.

Ladite charge de déshydratation est vaporisée dans un évaporateur. Après le passage dans l'évaporateur, il peut subsister une fraction non vaporisée. Cette fraction, liquide, est évacuée et constitue ledit effluent eau.

La fraction de la charge de déshydratation évaporée dans l'évaporateur est ensuite avantageusement chauffée et/ou comprimée aux conditions de température et de pression de la réaction de déshydratation.

L'effluent eau contient une fraction d'éthanol non réagit, qui sera recyclée après purification dans l'étape g), une fraction d'eau de dilution, et avantageusement les cendres et autres impuretés contenues dans l'éthanol. Cet effluent eau peut également contenir une fraction d'autres oxygénés, par exemple du diethylether issu d'une étape de prétraitement ou d'autres alcools lourds.

Ledit effluent éthylène (excluant l'eau) peut contenir jusqu'a 10000 ppm volumique de CO₂ et jusqu'à 15000 ppm volumique d'acétaldéhyde.

### Étape b) de condensation partielle

Conformément à l'invention, ledit effluent éthylène issu de l'étape a) de déshydratation subit un refroidissement suivi d'une condensation partielle par échange de chaleur avec au moins une source froide.

Dans cette étape du procédé et celles qui suivent, tous les flux contenant de l'eau liquide et des acides peuvent avantageusement être neutralisés à l'aide d'une solution aqueuse de soude. Le pH de la solution neutralisée doit être supérieur à 7 et avantageusement compris entre 7 et 9.

Préférentiellement, la charge de déshydratation est utilisée comme l'une de ces sources froide.

L'échangeur permet ainsi de condenser entre 50 et 95% poids de l'acétaldéhyde, préférentiellement entre 60 et 80% poids. Cet arrangement selon l'invention limite donc ainsi considérablement la quantité d'acétaldéhyde devant être traitée au cours de l'étape e).

La température de l'effluent éthylène partiellement condensé à l'issue de l'étape b) est choisie suffisamment basse pour que la majeure partie de l'acétaldéhyde compris dans ledit effluent éthylène partiellement condensé soit séparée dans ledit effluent liquide. La pression opératoire de l'étape c) correspond à celle de l'étape a) de déshydratation diminuée des pertes de charge entre les deux étapes. Elle se situe entre 0,2 et 1,1 MPa, préférentiellement entre 0,4 et 0,8 MPa.

L'échange de chaleur avec ladite source froide est tel que la température de l'effluent éthylène partiellement condensé issu de l'étape b) de condensation partielle est comprise entre 20 et 50°C, préférentiellement entre 25 et 45°C.

### Étape c) de séparation de phases

Conformément à l'invention, l'effluent éthylène partiellement condensé au cours de l'étape b) subit ensuite une étape de séparation de phases de manière à produire un effluent gaz et un effluent liquide.

### Étape d) de compression

Ledit effluent gaz issu de l'étape c) de séparation de phases subit ensuite une étape de compression de manière à produire un effluent gaz comprimé. Ladite compression peut être réalisée par tous moyens connus de l'homme du métier, par exemple en utilisant un compresseur centrifuge ou volumétrique, comprenant éventuellement plusieurs étages avec refroidissement intermédiaire.

La fraction liquide éventuellement condensée au cours de la compression subit avantageusement une étape g) de distillation.

Ladite étape de compression permet d'amener ledit effluent gaz comprimé à une pression suffisante pour permettre les étapes de purifications e), f) et g) et dont la pression opératoire est directement dépendante de ce compresseur, aux pertes de charge près. Ladite pression est comprise entre 1,1 et 5,1 MPa, de préférence entre 1,6 et 3,6 MPa.

Ledit effluent gaz comprimé est ensuite refroidi par échange de chaleur avec une source froide pour atteindre une température comprise entre 10 et 50°C, avantageusement entre 15 et 50°C, préférentiellement entre 25 et 45°C. Cette température est choisie la plus basse possible de manière à faciliter l'étape e) de lavage par solvatation des oxygéné.

### Étape e) de lavage

Conformément à l'invention, ledit effluent gaz comprimé refroidi subit ensuite une étape de lavage par la mise en contact avec de l'eau de lavage dans une colonne de lavage de manière à produire un effluent gaz lavé et un effluent eau usée.

Ladite étape de lavage permet de séparer les oxygénés (éthers, acides, aldéhydes, alcools) dudit effluent gaz comprimé refroidi. En particulier, ladite étape de lavage permet d'amener la concentration d'acétaldéhyde en dessous de 150 ppm poids, préférentiellement en dessous de 100 ppm poids. En effet, l'acétaldéhyde polymérise en milieu basique pour donner des huiles (huiles rouges, polyacétaldehydes) conduisant a minima à une détérioration des performances du lavage, et à terme, à l'inopérabilité de la colonne, par exemple en raison d'un bouchage des plateau ou du garnissage.

Ladite étape de lavage est mise en oeuvre dans une colonne de lavage comprenant des internes choisis parmi les garnissages structurés, les plateaux, ou toute autre technologie de mise en contact d'un gaz et d'un liquide connue de l'homme du métier.

Ladite eau de lavage comprend de l'eau, avantageusement en mélange avec de l'éthanol afin de faciliter la solubilisation des oxygénés, et potentiellement additivée d'agents stabilisants (bisulfites) ou d'antioxydants (alcools lourds, phénols, BHT). Le lavage des oxygénés est d'autant plus efficace que la température est basse (solvatation favorisée à basse température) et que la fraction d'éthanol dans l'eau de lavage est élevée. Cette fraction d'éthanol dans l'eau de lavage est comprise entre 0 et 50% poids, de préférence entre 0 et 10% poids.

Ladite étape de lavage opère à une température comprise entre 10 et 50°C, avantageusement entre 15 et 50°C, préférentiellement entre 25 et 45°C, ladite température étant ajustée lors de l'étape d) pour l'alimentation de la colonne, et lors de l'étape h) pour le fluide de lavage. Ladite température est choisie la plus basse possible en fonction des utilités disponible, avec la contrainte de rester supérieure a la température de formation des hydrates. On prendra une température supérieure à 10°C, avantageusement supérieure à15°C, préférentiellement supérieure a 25°C.

Préférentiellement, ledit effluent eau usée est traité dans l'étape g) de distillation de manière à séparer les oxygénés et permettre le recyclage de l'eau de lavage. Dans le cas ou la concentration en oxygénés (en particulier en acétaldehyde) dans ledit effluent eau usée est faible, c'est à dire au moins inférieure à la concentration en oxygénés dans l'effluent éthylène de l'étape a) de déshydratation), une fraction dudit effluent eau usée peut avantageusement être recyclée directement en amont de l'étape a) de déshydratation.

### Étape f) d'absorption

Le CO₂ est finalement séparé grâce à une colonne d'absorption à l'aide d'un solvant. Le gaz est purifié par mise en contact avec la solution absorbante, puis la solution absorbante est régénérée thermiquement. La composition de la solution absorbante est choisie pour sa capacité à absorber les composés acides sans interaction avec la matrice (oléfine).

Conformément à l'invention, ledit effluent gaz lavé subit une étape d'absorption par la mise en contact dans une colonne d'absorption avec au moins une solution absorbante de manière à produire un effluent gaz purifié et un effluent solution absorbante usée. Ladite étape d'absorption permet de séparer le CO₂.

La température de la (des) solution(s) absorbante(s) en entrée de l'étape f) d'absorption est comprise entre 25 et 50°C. La température de l'effluent gaz lavé est la température dudit effluent en sortie de l'étape e) de lavage.

La solution absorbante comprend un solvant choisi parmi les solvants chimiques et les solvants physiques.

Les solvants chimiques sont choisis parmi la soude et les amines choisies parmi la MEA (monoéthanolamine), la DEA (diéthanolamine), la MDEA (méthyldiéthanolamine), la DIPA (diisopropylamine), la DGA (diglycolamine), les diamines, la pipérazine et l'hydroxyéthyl piperazine prises seules ou en combinaison, par exemple en mélange d'une ou plusieurs amines tertiaires avec une ou plusieurs amines primaires ou secondaires. Si le solvant chimique choisi comprend de la soude, la solution absorbante comprend entre 10 et 50% poids de soude en solution aqueuse. Si le solvant chimique choisi comprend des amines, il comporte en général entre 10% et 80%, de préférence entre 20% et 60%, en poids d'amines, de préférence des alcanolamines et comportant au moins 20% poids d'eau, de préférence au moins 40% poids d'eau.

Les solvants physiques sont choisis parmi la N-formyl morpholine, des éthers de glycols, du sulfolane, du thiodiéthanol. Le solvant physique peut être mélangé avec un ou plusieurs solvant chimique mentionné ci-dessus et/ou avec de l'eau.

Ledit effluent solution absorbante usée peut être neutralisé ou recyclé. Dans le cas du recyclage de l'effluent solution absorbante usée, éventuellement après purification et/ou régénération dudit effluent solution absorbante usée, un appoint de solution absorbante fraiche pourra être effectué, afin de compenser les pertes en solution absorbante dans les différentes phases de l'étape f).

Par exemple, la soude sera neutralisée et de la soude fraiche sera importée pour alimenter ladite étape f) d'absorption. Dans le cas ou le solvant est une amine ou un solvant physique, il pourra être retraité et recyclé (ex situ ou in situ).

Ledit effluent gaz purifié est ensuite avantageusement compressé puis dirigé vers des étapes de purifications, par exemple il subira un séchage suivi d'une ou plusieurs distillation(s) cryogénique(s) qui permettront d'amener l'effluent gaz purifié aux spécifications recherchées pour l'éthylène. Cet effluent est le produit principal du procédé selon l'invention.

De manière optionnelle, Une masse de captation (régénérable ou non) du CO₂ pourra avantageusement être installée en fin de chaine afin d'éliminer les toutes dernière traces de CO₂ et, éventuellement, de diminuer le coût de traitement du solvant chimique ou physique.

### Étape g) de distillation

Conformément à l'invention, ledit effluent eau issu de l'étape a), ledit effluent liquide issu de l'étape c), et ledit effluent eau usée issu de l'étape e) subissent une étape de distillation dans une colonne à distiller de manière à produire au moins quatre coupes : un distillat vapeur, un distillat liquide, un soutirage intermédiaire, appelé effluent eau purifiée, contenant majoritairement de l'eau, l'éthanol non réagit condensé dans l'étape c) de séparation de phases, ainsi que l'éthanol compris dans l'effluent eau issu de l'étape a) de déshydratation, et un résidu de distillation, composé d'eau et de sels de résidu de neutralisation ou contenus dans l'alcool considéré. Ledit résidu de distillation représente la purge du procédé et son débit partiel en eau correspond au débit d'eau formée par la réaction de déshydratation.

La fraction liquide éventuellement condensée au cours de l'étape d) de compression subit également avantageusement l'étape g) de distillation.

Lesdits distillat vapeur et liquide contiennent en majorité l'acétaldéhyde extrait dans l'étape e) de lavage (10) et une fraction d'éthanol, d'éthylène, et d'eau.

Les conditions opératoires et le dimensionnement de la colonne sont tels que les pertes en éthanol et en éthylène soit les plus faibles possibles. La minimisation des pertes en éthylène conduit a avoir un reflux externe important, entre 1 et 10 fois le débit total des distillats liquide et vapeur, préférentiellement entre 3 et 7 fois. Le reflux externe correspond au flux liquide issu du ballon de reflux et recyclé en tête de la colonne de distillation.

Ladite colonne à distiller est opérée à une pression comprise entre 0,2 MPa et 1,1 MPa, préférentiellement entre 0,2 MPa et 0,4 MPa.

La dite colonne à distiller peut avantageusement être une colonne a paroi interne (dividing wall column dans la langue de Shakespeare).

La température du fond de ladite colonne est comprise entre 80 et 160°C, préférentiellement entre 100 et 150°C, et encore plus préférentiellement entre 120 et 140°C. La température du ballon de reflux de ladite colonne est comprise entre 20 et 50°C, préférentiellement entre 25 et 45°C.

### Étape h) de recyclage

Conformément à l'invention, ledit effluent eau purifiée est divisé en deux : une partie correspondant au débit d'eau de lavage nécessaire dans l'étape e) de lavage est refroidie par échange de chaleur avec une source froide et recyclée en amont de l'étape e) de lavage. Ladite partie est désignée sous le nom d'eau de lavage.

L'autre partie, désignée sous le nom d'eau de dilution, est avantageusement recyclée en amont de l'étape a) de déshydratation, de manière à servir de diluant thermique. Dans le cas où l'étape a) de déshydratation converti partiellement l'éthanol, l'eau de dilution contient du diethylether, non converti à l'issue de l'étape a) de déshydratation (cas des procédés de déshydratation à faible conversion par passe).

Conformément aux pratiques usuelles lors d'un recyclage, une purge est avantageusement réalisée sur le recyclage d'eau de lavage et/ou d'eau de dilution.

### Description des figures

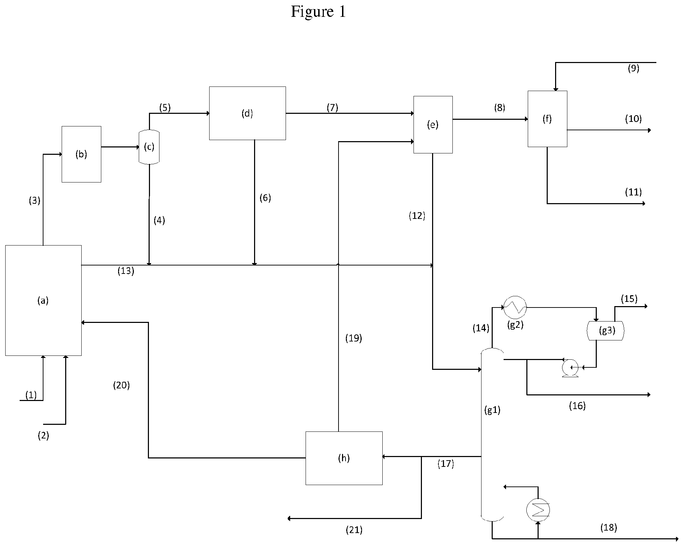

La figure 1 représente un arrangement possible du procédé selon l'invention.

La charge éthanol (1), l'eau de dilution (20) et un flux d'eau externe au procédé (2) alimentent l'étape (a) de déshydratation. L'effluent eau (13) est dirigé vers l'étape (g) de distillation. L'effluent éthylène (3) issu de l'étape (a) de déshydratation subit une étape (b) de condensation partielle. L'effluent de ce condenseur est amené dans une étape (c) de séparation des phases, où sont séparées un effluent gaz (5) et un effluent liquide (4).

L'effluent gaz (5) issu de l'étape (c) subit ensuite une étape (d) de compression. La fraction liquide (6) éventuellement produite peut avantageusement être mélangé à l'alimentation de l'étape (g) de distillation. L'effluent gaz comprimé (7) est ensuite conduit dans une étape (e) de lavage. L'effluent eau usée (12) est envoyé dans l'étape (g).

L'effluent gaz lavé (8) est amené dans l'étape (f) d'absorption, alimentée également par une solution absorbante (9). L'effluent solution absorbante usée (11) est soutiré de la colonne avant d'être neutralisé ou recyclé.

L'effluent gaz purifié (10) issu de ladite étape (f) constitue le produit principal du procédé.

Les effluent eau (13), effluent liquide (4), fraction liquide (6) et effluent eau usée (12) sont envoyés dans une étape (g) de distillation représentée sur la figure par les éléments (g1) à (g3). La tête (14) est partiellement condensée dans l'échangeur de chaleur (g2). Ce flux est ensuite introduit dans le ballon de reflux (g3) puis séparé en un reflux, un distillat vapeur (15) et un distillat liquide (16).

L'effluent eau purifiée (17) alimente l'étape (h) de recyclage. Une partie (19) est renvoyé vers l'étape (e) de lavage comme eau de lavage. L'autre partie (20) est l'eau de dilution, avantageusement recyclée vers l'étape (a) de déshydratation.

Le flux (21) représente un positionnement possible de la purge.

L' exemple suivant illustre l'invention sans en limiter la portée.

### Exemples

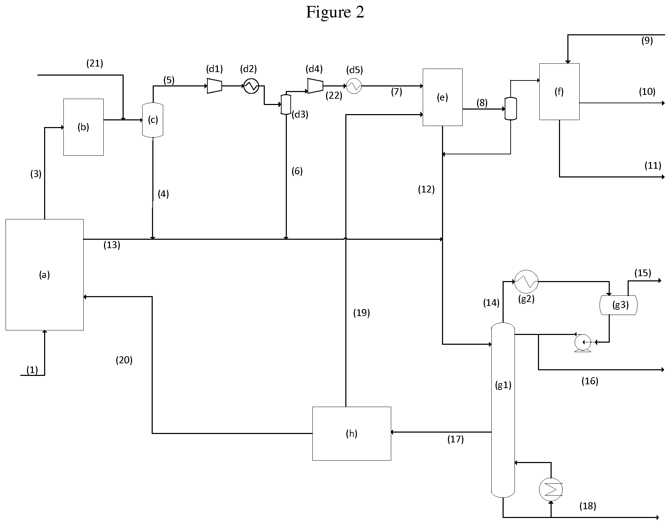

La figure 2 illustre l'exemple. La numérotation est identique à celle de la figure 1.

La figure 2 et le tableau 1 présentent un exemple de purification d'un effluent éthylène issu d'une étape de déshydratation. La charge de déshydratation est composée d'une charge éthanol (1) en mélange avec 66% poids d'eau de dilution (20), le pourcentage poids étant exprimé par rapport au poids total de ladite charge de déshydratation. Cette dilution permet de limiter la baisse de température due à l'endothermie de la réaction. La concentration en CO₂ en sortie du réacteur de déshydratation est de 1000 ppm volumique.

La charge éthanol considérée est produite par fermentation de blé, sans extraction des glutens, par un procédé de type dry milling selon le terme anglo-saxon. Cette charge est composée de 41671 kg/h d'éthanol en mélange avec 3993 kg/h d'eau et 9kg/h d'acétaldéhyde. La charge de déshydratation comprend également 103161 kg/h d'eau de dilution, ladite eau de dilution comprenant 3016 kg/h d'éthanol non réagit.

L'effluent éthylène (3) de la section réactionnelle est partiellement condensé dans une étape (b), puis neutralisé à l'aide d'une solution aqueuse de soude (21). Le pH du liquide (4) est fixé à 8.

La zone de compression est composée de deux étages (d1) et (d4) permettant de faire l'absorption sous une pression de 13 barg.

Le solvant d'absorption du CO₂ de la colonne (12) est une solution aqueuse de soude concentrée à 20% poids. Son débit est de 100kg/h.

Le tableau suivant donne les principales conditions opératoires:

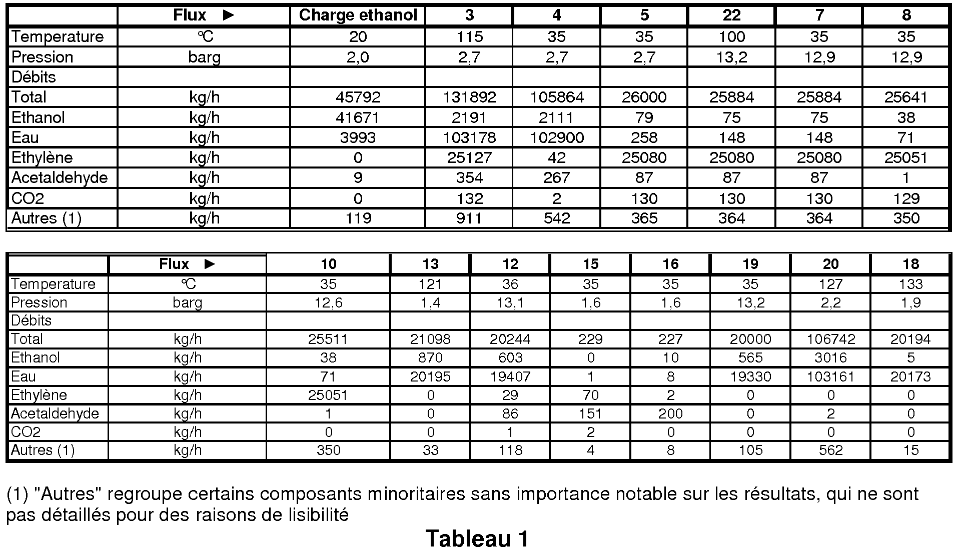

## Revendications

1. Procédé de production d'éthylène à partir d'une charge éthanol comprenant au moins les étapes suivantes :
a) Une étape de déshydratation d'une charge de déshydratation comprenant ladite charge éthanol de manière à produire au moins un effluent éthylène, et au moins un effluent eau,
b) Une étape de condensation partielle dudit effluent éthylène par échange de chaleur avec au moins une source froide à une température comprise entre 20 et 50°C,
c) Une étape de séparation de phases dudit effluent éthylène partiellement condensé de manière à produire un effluent gaz et un effluent liquide,
d) Une étape de compression dudit effluent gaz issu de l'étape b) de manière à produire un effluent gaz comprimé suivi d'un refroidissement à une température comprise entre 10 et 50°C,
e) Une étape de lavage par la mise en contact dudit effluent gaz comprimé avec l'eau de lavage recyclée selon l'étape h) dans une colonne de lavage de manière à produire un effluent gaz lavé et un effluent eau usée,
f) Une étape d'absorption par la mise en contact dudit effluent gaz lavé avec au moins une solution absorbante dans une colonne d'absorption, ladite solution absorbante comprenant au moins un solvant choisi parmi le groupe constitué par les solvants chimiques et physiques, de manière à produire un effluent gaz purifié et un effluent solution absorbante usée, lesdits solvants chimiques étant choisis parmi la soude et les amines choisies parmi la MEA (monoéthanolamine), la DEA (diéthanolamine), la MDEA (méthyldiéthanolamine), la DIPA (diisopropylamine), la DGA (diglycolamine), les diamines, la pipérazine et l'hydroxyéthyl piperazine prises seules ou en combinaison, lesdits solvants physiques étant choisis parmi la N-formyl morpholine, des éthers de glycols, du sulfolane, du thiodiéthanol,
g) Une étape de distillation dudit effluent eau usée issu de l'étape e) et, conjointement, dudit effluent eau issu de ladite étape de déshydratation de manière à produire au moins un distillat gaz, un distillat liquide, un effluent eau purifiée, et un résidu de distillation.
h) Une étape de recyclage d'au moins une partie dudit effluent eau purifiée issu de l'étape g) en amont de l'étape e) de lavage, ladite partie étant alors désignée sous le nom d'eau de lavage.

2. Procédé selon la revendication 1 dans lequel est ladite charge éthanol est une charge éthanol produite à partir de source renouvelable issue de la biomasse.

3. Procédé selon l'une des revendications 1 à 2 dans lequel ladite charge de déshydratation comprend également un appoint d'eau constitué de l'eau de dilution issue de l'étape h) de recyclage et/ou d'un flux d'eau d'origine externe au procédé.

4. Procédé selon l'une des revendications 1 à 3 dans lequel la charge de déshydratation est utilisée comme l'une des sources froide de ladite étape b).

5. Procédé selon l'une des revendications 1 à 4 dans lequel la température à l'issue de l'étape d) est comprise entre 15 et 50°C.

6. Procédé selon l'une des revendications 1 à 4 dans lequel la température à l'issue de l'étape d) est comprise entre 25 et 45°C.

7. Procédé selon l'une des revendications 1 à 6 dans lequel le solvant physique peut être mélangé avec un ou plusieurs solvants chimiques et/ou avec de l'eau.

8. Procédé selon l'une des revendications 1 à 7 dans lequel l'étape g) est mise en oeuvre dans une colonne à paroi interne.

## Patentansprüche

1. Verfahren zur Herstellung von Ethylen aus einer Ethanol-Charge, umfassend mindestens die folgenden Schritte:
a) einen Schritt der Dehydratisierung einer Dehydratisierungscharge, umfassend die Ethanol-Charge, um mindestens einen Ethylen-Abfluss und mindestens einen Wasserabfluss zu erzeugen,
b) einen Schritt der teilweisen Kondensation des Ethylen-Abflusses durch Wärmeaustausch mit mindestens einer Kältequelle bei einer Temperatur zwischen 20 und 50°C,
c) einen Schritt der Trennung der Phasen des teilweise kondensierten Ethylen-Abflusses, um einen Gasabfluss und einen Flüssigkeitsabfluss zu erzeugen,
d) einen Schritt der Kompression des Gasabflusses, der aus dem Schritt b) stammt, um einen komprimierten Gasabfluss zu erzeugen, gefolgt von einer Abkühlung auf eine Temperatur zwischen 10 und 50°C,
e) einen Schritt der Wäsche durch Inberührungbringen des komprimierten Gasabflusses mit dem rezyklierten Waschwasser gemäß Schritt h) in einer Waschsäule, um einen gewaschenen Gasabfluss und einen Abwasserabfluss zu erzeugen,
f) einen Schritt der Absorption durch Inberührungbringen des gewaschenen Gasabflusses mit mindestens einer Adsorptionslösung in einer Absorptionssäule, wobei die Absorptionslösung mindestens ein Lösungsmittel umfasst, ausgewählt aus der Gruppe bestehend aus chemischen und physikalischen Lösungsmittels, um einen gereinigten Gasabfluss und einen verbrauchten Absorptionslösungsabfluss zu erzeugen, wobei die chemischen Lösungsmittel aus Soda und Aminen ausgewählt werden, ausgewählt aus MEA (Monoethanolamin), DEA (Diethanolamin), MDEA (Methyldiethanolamin), DIPA (Diisopropylamin), DGA (Diglycloamin), Diaminen, Piperazin und Hydroxyethylpiperazin, allein oder in Kombination, wobei die physikalischen Lösungsmittel aus N-Formylmorpholin, Glycolethern, Sulfolan, Thiodiethanol ausgewählt werden,
g) einen Schritt der Destillation des Abwasserabflusses, der aus dem Schritt e) stammt, und gleichzeitig des Wasserabflusses, der aus dem Dehydratisierungsschritt stammt, um mindestens ein Gasdestillat, ein Flüssigkeitsdestillat, einen gereinigten Wasserabfluss und einen Destillationsrückstand zu erzeugen,
h) einen Schritt der Rezyklierung mindestens eines Teils des gereinigten Wasserabflusses, der aus dem Schritt g) stromaufwärts von dem Schritt e) der Wäsche stammt, wobei der Teil dann mit dem Namen Waschwasser bezeichnet wird.

2. Verfahren nach Anspruch 1, wobei die Ethanol-Charge eine Ethanol-Charge ist, die aus einer erneuerbaren Quelle aus Biomasse erzeugt wird.

3. Verfahren nach einem der Ansprüche 1 bis 2, wobei die Dehydratisierungscharge auch eine Zugabe von Wasser umfasst, die aus Verdünnungswasser, das aus dem Schritt h) der Rezyklierung stammt, und einem Wasserfluss verfahrensexternen Ursprungs besteht.

4. Verfahren nach einem der Ansprüche 1 bis 3, wobei die Dehydratisierungscharge als eine der Kältequellen des Schritts b) verwendet wird.

5. Verfahren nach einem der Ansprüche 1 bis 4, wobei die Temperatur am Ende von Schritt d) zwischen 15 und 50°C liegt.

6. Verfahren nach einem der Ansprüche 1 bis 4, wobei die Temperatur am Ende von Schritt d) zwischen 25 und 45°C liegt.

7. Verfahren nach einem der Ansprüche 1 bis 6, wobei das physikalische Lösungsmittel mit einem oder mehreren chemischen Lösungsmitteln und/oder mit Wasser gemischt werden kann.

8. Verfahren nach einem der Ansprüche 1 bis 7, wobei Schritt g) in einer Säule mit einer Innenwand durchgeführt wird.

## Claims

1. Process for the production of ethylene from an ethanol feedstock that comprises at least the following stages:
a) A stage for dehydration of a dehydration feedstock comprising said ethanol feedstock in such a way as to produce at least an ethylene effluent and at least a water effluent,
b) A stage for partial condensation of said ethylene effluent by heat exchange with at least a cold source at a temperature of between 20 and 50°C,
c) A stage for separation of phases of said partially condensed ethylene effluent in such a way as to produce a gas effluent and a liquid effluent,
d) A stage for compression of said gas effluent that is obtained from stage b) in such a way as to produce a compressed gas effluent followed by a cooling to a temperature of between 10 and 50°C,
e) A stage for washing by bringing said compressed gas effluent into contact with the recycled washing water according to stage h) in a washing column in such a way as to produce a washed gas effluent and a waste water effluent,
f) A stage for absorption by bringing said washed gas effluent into contact with at least an absorbent solution in an absorption column, with said absorbent solution comprising at least a solvent that is selected from among the group that consists of the chemical and physical solvents, in such a way as to produce a purified gas effluent and a waste absorbent solution effluent, said chemical solvents being selected from among soda and the amines are selected from among MEA (monoethanolamine), DEA (diethanolamine), MDEA (methyl diethanolamine), DIPA (diisopropylamine), DGA (diglycolamine), diamines, piperazine, and hydroxyethyl piperazine, taken by themselves or in combination, said physical solvents being selected from among N-formyl morpholine, ethers of glycols, sulfolane, and thiodiethanol,
g) A stage for distillation of said waste water effluent that is obtained from stage e), and, jointly, said water effluent that is obtained from said dehydration stage in such a way as to produce at least a gas distillate, a liquid distillate, a purified water effluent, and a distillation residue,
h) A stage for recycling at least a portion of said purified water effluent that is obtained from stage g) upstream from the washing stage e), with said portion then being referred to as washing water.

2. Process according to Claim 1, in which said ethanol feedstock is an ethanol feedstock that is produced from a renewable source that is obtained from the biomass.

3. Process according to one of Claims 1 to 2, in which said dehydration feedstock also comprises an addition of water that consists of the dilution water that is obtained from the recycling stage h) and/or a stream of water from a source that is external to the process.

4. Process according to one of Claims 1 to 3, in which the dehydration feedstock is used as one of the cold sources of said stage b).

5. Process according to one of Claims 1 to 4, in which the temperature at the end of stage d) is between 15 and 50°C.

6. Process according to one of Claims 1 to 4, in which the temperature at the end of stage d) is between 25 and 45°C.

7. Process according to one of Claims 1 to 6, in which the physical solvent can be mixed with one or more chemical solvents and/or with water.

8. Process according to one of Claims 1 to 7, in which stage g) is implemented in a column with an inside wall.
